# EUROPEAN PATENT SPECIFICATION

(11) **EP 3 356 973 B1**
(45) Date of publication and mention of the grant of the patent: **27.12.2023**
(21) Application number: 16849909.3
(22) Date of filing: 13.09.2016
(51) Int. Cl.: G16H 20/30, G16H 10/60

(54) **PORTABLE MEDICAL APPARATUS FOR INDIVIDUALIZED TREATMENT GUIDANCE BASED ON AN ONLINE STORAGE PROFILE WITH BODY MEASUREMENTS AND METHOD**
TRAGBARE MEDIZINISCHE VORRICHTUNG ZUR ANLEITUNG EINER INDIVIDUALISIERTEN BEHANDLUNG AUF DER GRUNDLAGE EINES ONLINE-SPEICHERPROFILS MIT KÖRPERMESSUNGEN UND VERFAHREN
APPAREIL MÉDICAL PORTATIF POUR GUIDAGE DE TRAITEMENT INDIVIDUALISÉ BASÉ SUR UN PROFIL DE STOCKAGE EN LIGNE AVEC MESURES CORPORELLES ET PROCÉDÉ

(30) Priority: 28.09.2015 US 201562233476 P
(43) Date of publication of application: 08.08.2018
(73) Proprietor: Koninklijke Philips N.V., 5656 AG Eindhoven (NL)
(72) Inventor: CHAO, Pei-Yin, 5656 AE Eindhoven (NL); KNOESTER, Jaap, 5656 AE Eindhoven (NL); DJAJADININGRAT, Johan Partomo, 5656 AE Eindhoven (NL); KIM, SeYoung, 5656 AE Eindhoven (NL); VAN LEENGOED, Marleen Johanna Jacoba, 5656 AE Eindhoven (NL); LUI, Wing Lam, 5656 AE Eindhoven (NL)
(74) Representative: Philips Intellectual Property & Standards
(86) International application number: PCT/IB2016/055431
(87) International publication number: WO 2017/055953

(56) References cited:
- US-A1- 2015 170 546
- US-A1- 2015 193 583

## Description

The present embodiments relate generally to portable medical apparatus and more particularly, to portable medical apparatus for individualized treatment guidance based on an online storage profile with body measurements and a method thereof.

Cardio-Pulmonary Resuscitation (CPR) quality is important in saving a person's life in case of a Sudden Cardiac Arrest (SCA), as promulgated by the American Heart Association (AHA) and the European Resuscitation Council. To deliver high-quality CPR, rescuers need to act with the right depth (i.e., compression of the victim's chest) and speed (i.e., frequency of compression). Guidelines recommend a depth of two inches (2 in). Compressions move blood through the body to keep vital organs oxygenated. In addition, adequate depth essentially traps the heart between the sternum and spine and effectively squeezes the blood out. Otherwise, inadequate CPR occurs.

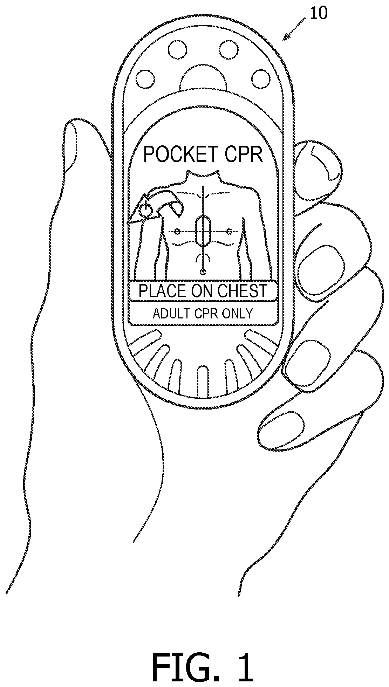

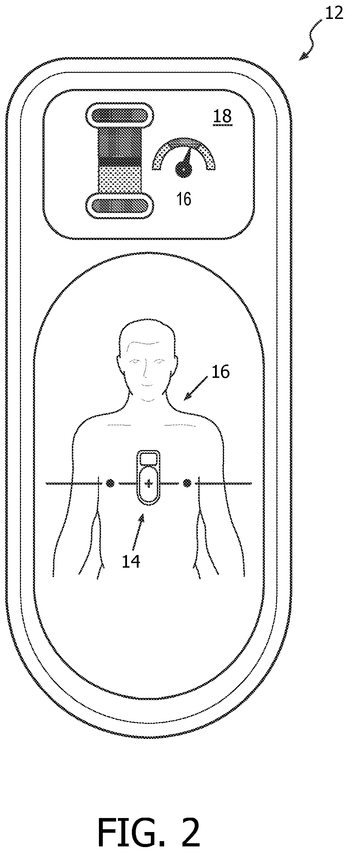

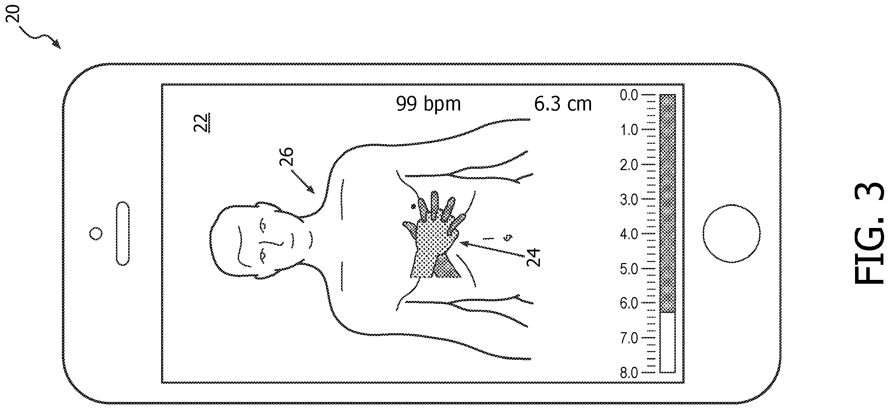

There are several known CPR metronomes that guide a rescuer in administering the "correct" depth of compression on a victim's chest; however, a disadvantage of each is that they assume a fixed depth for compression, regardless of the victim's body measurements. Figure 1 shows an image view of a CPR metronome device 10 known as a ZOLL^{™} PocketCPR^{™}. The CPR metronome device 10 includes on a front surface thereof an illustration of the device and its placement upon a victim's chest while administering CPR. Figure 2 shows an image view of another CPR metronome device 12 known as a Philips^{™} CPR metronome. The CPR metronome device 12 of Figure 2 includes on a front surface thereof an illustration of the device, indicated by reference numeral 14, and its placement upon a victim's chest, indicated by reference numeral 16, while administering CPR. The device 12 of Figure 2 further includes a display 18 configured to indicate a CPR parameter, e.g., a timing of compressions. Figure 3 is a screen display view of a smart phone 20 running a CPR app 22 that includes a general representation of an approximate placement of a user's hands 24 on a victim's chest 26. However, currently known CPR metronomes guide rescuers in deploying a same compression depth for thin as well as large people. A problem exists in that deploying too little depth is ineffective and deploying too much depth potentially leads to damage to structures being compressed, for example, especially with respect to CPR chest compression depth in children. Also, rib fracture is known to happen during CPR, which in turn causes other complications.

In other words, one problem in administering CPR is the use of chest compressions that are too deep or too shallow. For example, when a victim's chest is compressed too much during CPR, the victim's ribcage may fracture. A fractured ribcage takes anywhere from 6 weeks, up to 12 weeks, to heal. During this period of time, the victim can experience severe pain when breathing, laughing, coughing, sneezing and/or changing posture. In addition, sharp rib fractures can cause a pneumothorax or bleeding lung, leading to shortness of breath and severe pain. In some severe cases, a fractured ribcage can even damage the liver, kidneys and/or spleen.

Another problem in administering CPR is incomplete chest release. While the depth of compression, i.e.., compressing the chest deep enough, is important, it is also important to release the pressure on the victim's chest sufficiently. If the first responder does not raise his hands sufficiently, the victim's chest remains compressed and the pumping effect on the heart is diminished.

In addition to CPR for emergency care, automatic external defibrillators (AEDs) are also known. With the use of an AED, resuscitation must be done according to a strict and time critical protocol. Typically, resuscitation of a victim is started by an informal caregiver (e.g., a colleague, a family member, a bystander) since the first minutes of the emergency are crucial and it will take time for someone to arrive with an AED. For resuscitation (i.e. pumping action on the victim's chest, alternated with mouth-to-mouth) to function correctly, the informal caregiver needs to act with the right depth (i.e., compression of the victim's chest) and speed (i.e., frequency of compression).

Further in connection with defibrillation and emergency care with an AED, it is noted that when a heart is fibrillating, disorganized electrical signals disrupt the synchronized contraction of the heart muscles. The AED device can help to bring the heart's electrical signals back in sync by administering an electric shock. To administer this shock, two adhesive electrode pads need to be positioned onto the victim's body in certain locations and orientations. In particular, the electrode pads need to be put along a body diagonal with the heart located in between the electrode pads. An optimal placement for an adult patient is as follows. The first pad is attached to the upper right chest, then the second pad is attached to the lower left side of the victim. For a professional caregiver this may be easy, but for an informal caregiver this can be tricky. Some AEDs may provide a step-by-step verbal instruction and/or illustrations that assist an informal caregiver to correctly use the electrode pads and the device. However, for a lay person guided only by an illustration on the electrode pads themselves, it may be difficult to put the pads in an optimal location on a victim needing emergency care.

Thus, in addition to the problems discussed above with respect to administering CPR, one problem in using an AED is incorrect electrode pad placement on a victim. For optimal defibrillation the two electrode pads should be placed correctly on the victim's body. Medical professionals know how to place the pads relative to body features such as nipples, belly button and rib lines. However, a layman first responder does not know this information and it is hard for a product to communicate this complex medical information in an emergency situation as the 2D graphics usually found illustrated on each electrode pad are difficult to relate to the victim's body.

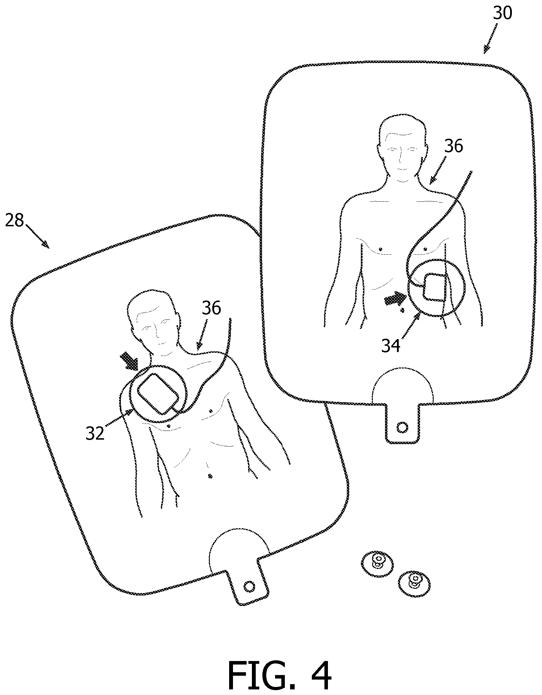

With reference to Figure 4, an illustrative view is shown of two AED electrodes, indicated by reference numerals 28 and 30, having indications thereon that feature a general representation, indicated by reference numerals 32 and 34, for an approximate placement of the electrodes on a victim's chest 36. In other words, Figure 4 illustrates two AED electrode pads with graphics for instructing the first responder where to place the pads. It can be difficult to relate this generic picture to the victim at hand in an emergency situation.

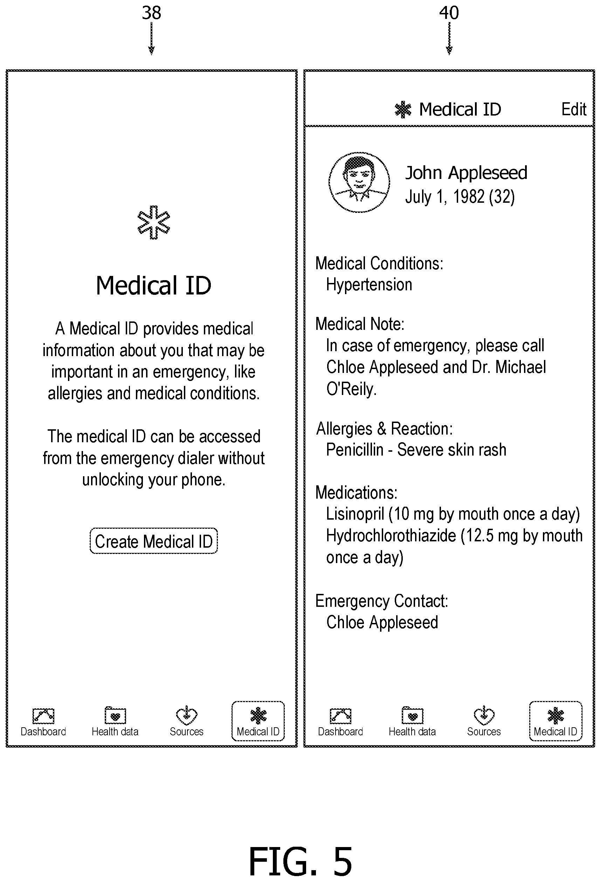

With reference now to Figure 5, an example of two screen display views, indicated by reference numerals 38 and 40, of a medical ID or health medical passport app operating on a smartphone is illustrated. The medical ID or health medical passport app includes use of a user profile to provide medical information about the user on the user's smartphone (e.g., Apple^{™} Health). As noted in screen display view 38, the medical information includes, for example, allergies and medical conditions, which may be important in the event of an emergency or accident. In addition, the health medical passport app on the smartphone is accessible without having to unlock the phone. An example profile is shown in screen display view 40 and can include a user's name, date of birth, medical conditions, medical note, allergies and reaction, medications, and an emergency contact. However, the medical ID or health medical passport app and the user's medical information are not tailored, or specific, to a SCA event. In addition, the user's smartphone may not be readily available or accessible during an emergency or accident.
US 2015/0170546 A1 discloses a method for guiding a user in performing a Cardio-Pulmonary Resuscitation (CPR) procedure on a patient involving a user portable device comprising a camera, and a display. The method includes receiving a video of the patient captured by the camera, and processing the video to segment a chest region of the patient. A target position on the chest where to position hands for performing CPR is then determined, and this position is then shown on the display, hereby guiding the user in obtaining a suitable hand position for performing CPR. The method can be implemented as a software application in a personal portable device such as a smartphone, a tablet application software, a wearable computer with headmounted display etc. Further, the video of the scene captured by the camera can be processed to provide information regarding functional quality of CPR, e.g. compression frequency and depth, during the CPR procedure. Further, vital signs of the patient such as heart rate, respiration rate, and blood oxygen saturation may be derived by image processing on the video, i.e. without any dedicated medical sensors. All such information can be provided as visual and/or audible feedback to the user during the CPR procedure, thus improving CPR effectiveness, also in case of an untrained user.

Every second counts during cardiac arrest. Accordingly, it would be desirable to provide more advanced AEDs that could cut down on the time needed to start resuscitation, thus improving the chances of survival for a victim. In addition, an improved method and apparatus for overcoming the problems in the art is desired.

The invention is defined by the independent claims of the appended set of claims.
Figure 1 is an image view of a known CPR metronome device;
Figure 2 is an image view of another known CPR metronome device;
Figure 3 is a screen display view of a smart phone running a CPR app that includes a general representation of an approximate placement of a user's hands on an victim's chest;
Figure 4 is an illustrative view of two AED electrodes having indications thereon that feature a general representation for an approximate placement of the electrodes on a victim's chest;
Figure 5 illustrates two screen display views of a medical ID or health tracking app operating on a smart phone;
Figure 6 is a flow diagram view of a method for real-time individualized treatment guidance for a subject using a portable medical device according to an embodiment of the present disclosure;
Figure 7 is a block diagram view of a portable medical apparatus for real-time individualized treatment guidance for a subject according to an embodiment of the present disclosure;
Figure 8 is an image view of the portable medical apparatus being used by a user for real-time individualized treatment guidance for a subject according to an embodiment of the present disclosure;
Figure 9 is another image view of the portable medical apparatus being used by the user for real-time individualized treatment guidance for the subject, including overview guidance of individualized placement of AED electrodes, according to an embodiment of the present disclosure;
Figure 10 is another image view of the portable medical apparatus being used by the user for real-time individualized treatment guidance for the subject, including overview guidance of individualized compression via the user's hands for CPR, according to an embodiment of the present disclosure;
Figure 11 is another image view of the portable medical apparatus being used by the user for real-time individualized treatment guidance for the subject, including overview guidance of individualized placement of the user's hands for CPR, according to an embodiment of the present disclosure;
Figure 12 is a block diagram view of a portable medical apparatus for real-time individualized treatment guidance for a subject according to another embodiment of the present disclosure;
Figure 13 is an image view of an ECG lead unit interface box for a portable medical apparatus having indications thereon that feature a general representation for an approximate placement of electrodes on a subject's body, the interface box further having cables or leads with electrodes or sensors (not shown) at distal ends thereof for use by a care provider during real-time individualized treatment guidance for the subject, according to an embodiment of the present disclosure;
Figure 14 is an illustrative diagram view of ECG electrode placement on a subject's body; and
Figure 15 is a flow diagram view of a method for real-time individualized treatment guidance for a subject using a portable medical device according to another embodiment of the present disclosure.

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein can be used in the practice or testing of the embodiments.

As indicated herein above, in accordance with one aspect, a portable medical apparatus for real-time individualized treatment guidance for subjects with diverse body measurements is disclosed. The portable medical device advantageously guides a first responder or rescuer in administering optimal CPR and the best use of an AED through use of the victim's bodily measurements. By tailoring the guidance on compression depth and release to a victim's body measurements, a rescuer can administer high-quality CPR whilst minimizing the chance of injuring the victim's ribcage. By knowing the relevant chest dimensions of the victim, the rescuer has an improved opportunity and greater chance of attaching the electrode pads in exactly the right locations on the victim's body.

During an emergency, one or more problems arise with respect to taking body measurements of the victim. Ideally, during an emergency, it would be desirable to have some kind of vision system (e.g. using the camera in a head-mounted display) which can visually discern the critical bodily dimensions, based on recognition of features such as nipples, belly button and rib lines. However, it is difficult to obtain robust automatic recognition considering the complexities of a real-life situation, including male and female victims, occlusion of distinguishing features through partially removed clothing, sagging body parts and body hair.

In case of a Sudden Cardiac Arrest (SCA), Cardio-pulmonary Resuscitation (CPR), i.e. a pumping action on the victim's chest, needs to be immediately administered in order to save the victim's life. As soon as an Automatic External Defibrillator (AED) arrives on the scene, a defibrillating shock may need to be administered. Essential in administering quality CPR are (i) pressing down on the right location on the victim's chest (i.e., the sternum); and (ii) deployment of correct compression rate, depth and complete release on the victim's chest. Essential in administering quality defibrillation is correct positioning of the electrode pads. The three actions of pressing down, deployment of correct compression depth and release, and positioning of AED electrode pads have in common that they depend on the body measurements of the victim.

According to one embodiment, the portable medical device and method advantageously use access to online storage of a personal profile which contains the victim's bodily measurements. In an emergency, this online profile may be accessed by the portable medical apparatus which can include one or more different devices (e.g. a CPR metronome, a CPR app, an augmented reality headset providing guidance) in order to provide victim-optimized CPR guidance to the first responder.

According to another embodiment, the portable medical device makes use of a cloud-based health record, and more particularly, a person's online health record which includes those body dimensions which are critical for optimized emergency care. In case of an emergency, the body and physical dimension information can advantageously be downloaded by the portable medical device to provide victim-tailored guidance so that the first responder or rescuer does not need to guess while administering an emergency treatment.

A person's online health record, or cloud-based health record, include one or more types of measurements, and more particularly, at least a subset of a person's bodily dimensions. Measurements and/or bodily dimensions can be obtained, for example, using a tape measure or via a more advanced methods, e.g., with a 3D body scanner. According to one embodiment, the method includes storing bodily measurements in a unique personal online profile. During an emergency, the method includes identifying the victim in order to download his/her body measurements from his/her online profile. During the emergency, the method further includes actively adjusting the guidance provided to the first responder or rescuer from the portable medical device, for example, including a CPR metronome or head-mounted augmented reality (AR) display, based on the downloaded body measurements of the victim.

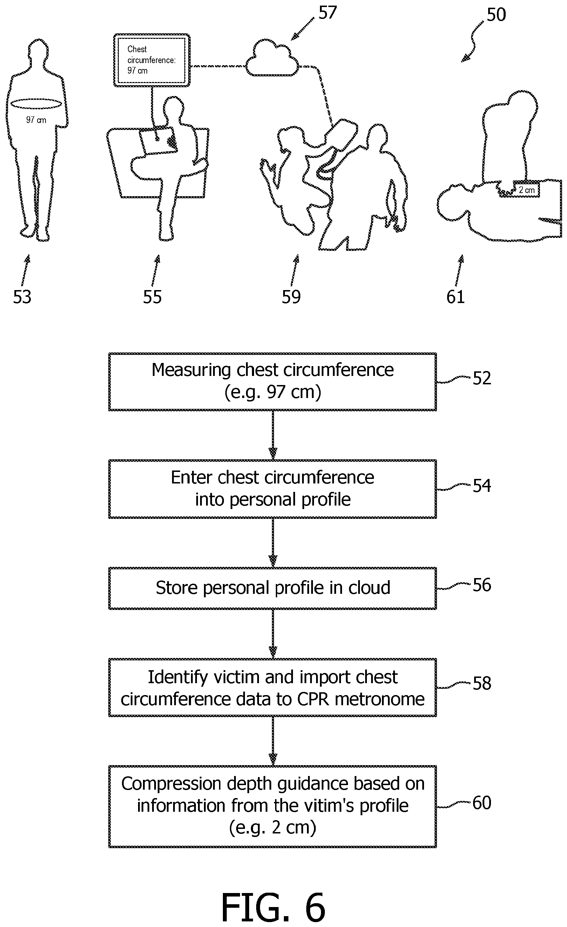

With reference now to Figure 6, there is shown a flow diagram view of a method 50 for real-time individualized treatment guidance for a subject using a portable medical device according to an embodiment of the present disclosure. The method comprises a first step 52 of measuring a critical body dimension of the subject, e.g., measuring chest circumference, as is also illustrated pictorially at 53. For example, the subject's chest circumference could be 97 cm (or 38 inches). The second step 54 includes entering the measured body dimension of the subject into a personal profile, as is also illustrated pictorially at 55. The third step 56 includes storing the profile in online storage or the cloud, as is also illustrated pictorially at 57. The fourth step 58 includes identifying the victim and importing body dimension data to the portable medical device, e.g., a CPR metronome, as is also illustrated pictorially at 59. In a fifth step 60, the method includes providing compression depth guidance based on information from the victim's profile, as is also illustrated pictorially at 61. For example, the compression depth guidance may be 5 cm (i.e., approximately 2.0 inches).

In a first aspect, the method comprises measuring the critical body dimensions of a subject. Cardiovascular patients at risk of a sudden cardiac arrest, but potentially everyone, would need to have their bodily dimensions measured, give appropriate permission to store these dimensions in a personal online profile and allow for the stored dimensions to be automatically downloaded in case of an emergency. As previously mentioned, bodily dimensions could simply be measured with a tape measure or, via a more advanced method, e.g., with a 3D body scanner. In one embodiment, the physical body dimensions could be measured and/or obtained by a general practitioner (GP), at a health centre or in a hospital lab.

According to the embodiments of the present disclosure, access to bodily dimensions, that include at least (i) chest depth, (ii) distance from eyes to the sternum, and (iii) optimal AED electrode positions relative to the face, are advantageously used by the portable medical device for providing victim-tailored guidance in the delivery of optimal CPR and/or other emergency treatment. With respect to chest depth, which corresponds to a dimension from the top of the sternum to the back of the spine, the victim-tailored guidance includes providing, via suitable means of the portable medical device to the first responder or rescuer as discussed further herein, an optimal chest compression depth (e.g., less compression for a skinnier person, more compression for a fatter person). In addition, with respect to the distance from the eyes to the sternum, the victim-tailored guidance includes providing, via suitable means of the portable medical device to the first responder or rescuer, e.g., through an augmented reality overlay as discussed further herein, where he or she should put his or her hands for optimal CPR. The face is currently the most easy to detect part of the body for computer vision systems and therefore forms a useful reference point. Furthermore, with respect to optimal AED electrode positions relative to the face, i.e., whether in 2 or 3 dimensions, the victim-tailored guidance includes providing via suitable means of the portable medical device to the first responder or rescuer, e.g., through an augmented reality overlay as discussed further herein, showing to the first responder or rescuer where he or she should place the adhesive electrode pads for optimal defibrillation.

In a second aspect, the method comprises storing the body measurements in a unique personal online profile. The above mentioned bodily dimensions are preferably stored in a profile accessible to the patient and care providers. This profile may be part of at least one of (i) a database especially set-up for SCA emergencies, (ii) a profile in a health tracking app on a patient's smartphone, and (iii) a person's Electronic Patient Record (EPR). As discussed herein above, Figure 5 illustrates an example of two screen display views of a medical ID or health medical passport app operating on a smartphone. The health medical passport app on the smartphone is accessible without having to unlock the phone. According to one embodiment, such a health medical passport app also includes body dimensions relevant to SCA.

In a third aspect, the method comprises identifying the victim in an emergency situation. In order to download the victim's body measurements, the victim is first identified. According to one embodiment of the present disclosure, the method and portable medical device apparatus advantageously incorporate use of at least one of a smartphone, a CPR metronome, and a head-mounted device (e.g., Google^{™} glass or augmented reality (AR) glasses) configured to identify the victim via one or more of performing face recognition and taking ID information carried by the victim. In connection with face recognition, the method and portable medical device includes an app that searches through an online database for a record with a face which matches the face of the victim. With respect to taking ID information carried by the victim, the method and portable medical device make use of one or more of an ID number, a QR code, or a near-field communication (NFC) chip. The victim could also carry such information on a tag with medical information (e.g., necklace, bracelet), on his or her phone (e.g., on the 'unlock' screen so that the first responder doesn't need an access code), as an implant, or as a tattoo. Advantageously, a tattoo may not only function as a means for identification, but also as a reference marker, e.g., a known point of origin, on the victim's body. The method includes, operating via the portable medical device, in response to first responder carrying out one or more of the following: entering the ID number into the portable medical device via an input/output device, capturing the QR code via a camera-based device, and scanning the NFC chip.

In a fourth aspect, the method comprises adjusting the victim-tailored guidance provided by the portable medical device. In particular, the method comprises adjusting the victim-tailored guidance based on the victim's body dimensions obtained from the online profile, wherein responsive to the obtained body dimensions, the portable medical device adjusts the victim-tailored guidance it provides for treatment during an emergency. According to the embodiments of the present disclosure, the method and portable medical device include one or more of (i) an accelerometer-based CPR metronome, (ii) an accelerometer-equipped smartphone with a CPR app, and (iii) a head-mounted augmented reality headset.

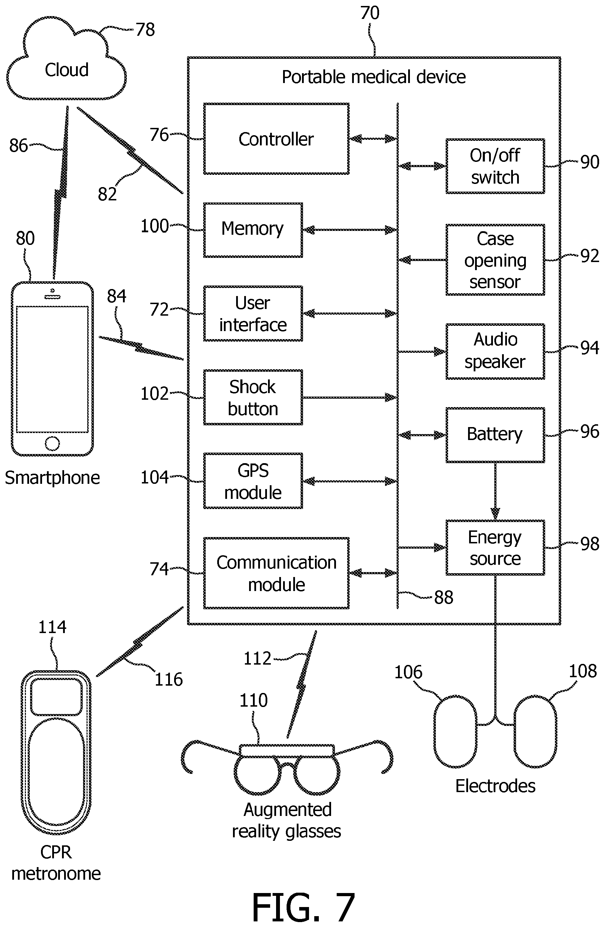

With reference now to Figure 7, a block diagram view of a portable medical apparatus 70 for real-time individualized treatment guidance for a subject according to an embodiment of the present disclosure is shown. The portable medical apparatus 70 comprises at least a user interface 72, a communications module 74, and a controller 76. In one embodiment, the user interface 72 is configured for at least obtaining the subject's identification information. The user interface 72 can comprise any suitable user interface operatively coupled to at least the controller 76, via signal lines 88, for use in connection with a given treatment during an emergency, as discussed further herein. For example, user interface 72 can comprise at least one selected from the group consisting of an input/output device, a tactile output device, a touch screen, an optical display, a microphone, a keypad, a keyboard, a pointing device, an image capture device, a video camera, an audio output device, and any combination thereof, determined as appropriate according to the requirements of a given portable medical device implementation and/or application.

The communications module 74 is configured for communicating with at least one of (i) a remote server 78 (i.e., also referred to herein as the cloud) having stored thereon a cloud-based electronic health record that comprises at least the subject's physical body measurements and (ii) a smartphone 80 located within a given immediate proximity of the portable medical apparatus. The communications module 74 is further for retrieving, via at least one of the remote server 78 or the smartphone 80 communicating with the remote server, in response to the subject's identification information, the cloud-based electronic health record stored on the remote server 78 (i.e., stored in the cloud).

Communication between the communication module 74 of the portable medical device 70 and the cloud 78 is indicated by reference numeral 82. Communication between the communication module 74 of the portable medical device 70 and the smartphone 80 is indicated by reference numeral 84. Lastly, communication between the smartphone 80 and the cloud 78 is indicated by reference numeral 86. Communication between the various devices and components as discussed herein is preferably accomplished using suitable techniques known in the art, and thus are not discussed further herein.

The controller 76 operatively couples to the user interface 72 and the communication module 74 via suitable signal lines, indicated via reference numeral 88. Controller 76 is configured for generating, in response to at least the subject's physical body measurements from the cloud-based electronic health record, real-time individualized treatment guidance to be followed by a user within the given immediate proximity of the portable medical apparatus 70 for administering the individualized treatment to the subject. In one embodiment, controller 76 comprises one or more of a microprocessor, microcontroller, field programmable gate array (FPGA), integrated circuit, discrete analog or digital circuit components, hardware, software, firmware, or any combination thereof, for performing various functions as discussed herein, further according to the requirements of a given portable medical device implementation and/or application. Controller 76 can further comprise one or more of the various modules as discussed herein. Additional details regarding the controller 76 will be provided herein below with reference to the Figures.

With reference still to Figure 7, the portable medical apparatus 70 can further comprise one or more of an ON/OFF switch 90, a case opening sensor 92, an audio speaker 94, a battery 96, an energy source 98, memory 100, shock button 102 (e.g., for activating the administration of a shock via AED pad electrodes), and GPS module 104. Each of the one or more of the ON/OFF switch 90, case opening sensor 92, audio speaker 94, battery 96, energy source 98, memory 100, shock button 102, and GPS module 104 is operatively coupled to at least the controller 76, e.g., via signal lines 88. The ON/OFF switch 90 comprises any suitable switch for powering the portable medical apparatus 70 between ON and OFF. The case opening sensor 92 comprises any suitable sensor for sensing the opening of the case or housing of the portable medical apparatus 70, for example, as part of initiating a start-up sequence for the portable medical apparatus. Audio speaker 94 can comprise any suitable speaker for a given implementation and/or portable medical device application.

In one embodiment, battery 96 can comprise any suitable power source or power supply for a given portable medical device implementation and/or application. In addition, energy source 98 can comprise any suitable power source or power supply for a given portable medical device implementation and/or application. For example, for a portable medical device comprising an AED device, the power source 98 can comprise a rechargeable power source. The power source 98 could also comprise a power supply via a source external to the portable medical device 70 or from a non-rechargeable power source. Furthermore, memory 100 can comprise any suitable memory device, operatively coupled to at least the controller 76, for at least storing information thereto, and further for at least subsequently retrieving the information there from.

The global positioning system module 104 comprises any suitable GPS module configured for determining a global position of the portable emergency medical apparatus 70. The controller 76 is further configured for determining that the smartphone is located within a given immediate proximity of the portable emergency medical apparatus based on the global position of the portable emergency medical apparatus 70 and a global position of the smartphone 80.

The portable medical apparatus 70 can further comprise a pair of AED pad electrodes 106,108 operatively coupled to energy source 98, for administration of an electrical shock during use of the portable medical device 70 as an AED device. The portable medical apparatus 70 can further comprise a pair of augmented reality glasses 110 to be worn by a first responder or rescuer, e.g., in performing AED and/or CPR during an emergency treatment. The augmented reality glasses 110 are operatively coupled via suitable communication link 112 (e.g., a near field communication (NFC), Bluetooth^{™}, or other suitable short-range communication link) with communication module 74 of the portable medical apparatus 70. Still further, the portable medical apparatus 70 can comprise a CPR metronome 114 to be used by a first responder or rescuer in performing CPR during an emergency treatment. The CPR metronome is operatively coupled via suitable communication link 116 (e.g., a near field communication (NFC), Bluetooth^{™}, or other suitable short-range communication link) with communication module 74 of the portable medical apparatus 70.

With reference now to Figures 6 and 7, in one embodiment, the CPR metronome 114 comprises an accelerometer-based CPR metronome. The CPR metronome is configured to take into account chest depth to guide the first responder on how deep to press, as illustrated in Figure 6 and indicated at reference numerals 60 and 61. In particular, the portable medical device 70 accesses the victim's cloud-based medical record, as discussed herein, and obtains or downloads the previously taken chest measurement from the victim's online record, such that use of the CPR metronome 114 for administering CPR takes into account the victim's chest depth as provided in real-time treatment guidance to the first responder or rescuer during the emergency treatment, further via user interface 72, for example, through information on a display or through audio guidance. In another embodiment, the CPR metronome 114 can comprise an accelerometer-equipped smartphone with a CPR app which takes into account chest depth to guide the first responder on how deep to press, either through information on a display or through audio guidance. Note that while the above makes reference to accelerometer-based metronome devices, such devices may not always need to be accelerometer-based. Essentially, the metronome device is depth-aware relative to the body. Accordingly, in another embodiment, augmented reality glasses can be used to establish a position of the CPR metronome by other means than an accelerometer, for example, using computer-vision.

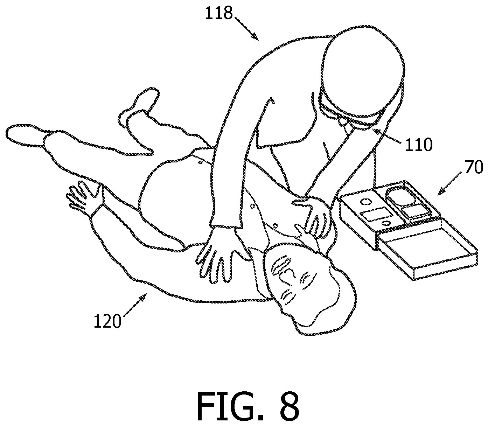

With reference now to Figures 7 and 8, in one embodiment, the augmented reality glasses 110 can comprise a head-mounted augmented reality headset. The head-mounted augmented reality glasses are configured to take into account body measurements to guide the first responder or rescuer 118 through an emergency procedure or treatment of a victim 120. For example, the portable medical device 70 accesses the victim's cloud-based medical record, as discussed herein, and obtains, i.e., downloads, the previously taken chest measurement from the victim's online record, such that use of the head-mounted augmented reality headset 110 and the CPR metronome 114 for administering CPR takes into account the victim's chest depth as provided in real-time treatment guidance to the first responder or rescuer 118 during the emergency treatment. In other words, the real-time treatment guidance takes into account chest depth to guide the first responder on how deep to press.

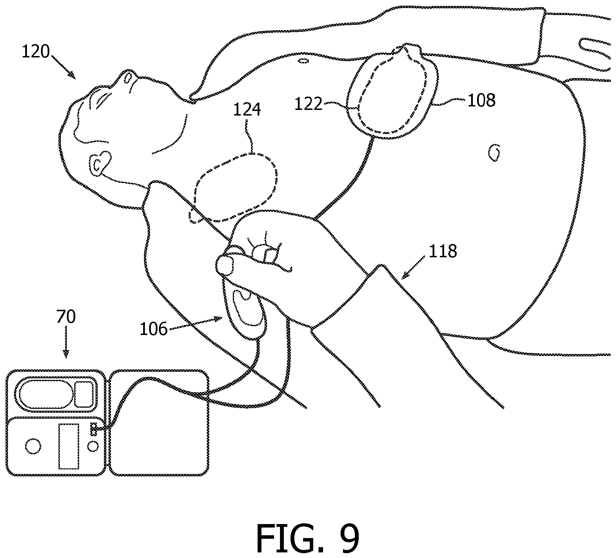

With reference now to Figures 7, 8 and 9, in one embodiment, the head-mounted augmented reality glasses 110 are configured detect the victim's face and to further take into account the victim's body dimensions to point out to the rescuer 118 optimal location and orientation to apply each of the AED electrode pads 106 and 108 on the victim's chest for the emergency treatment, i.e., AED. For example, the portable medical device 70 accesses the victim's cloud-based medical record, as discussed herein, and obtains, i.e., downloads, the previously taken bodily measurements from the victim's online record. Accordingly, the head-mounted augmented reality headset 110 is used for guiding a placement of the AED pad electrodes for administering AED which takes into account the bodily measurements of the victim as provided in the real-time treatment guidance to the first responder or rescuer 118 during the emergency treatment. In this embodiment, the head-mounted augmented reality headset 110 points out the exact location and orientation for the first responder to put each AED pad electrode, based on the victim's previously taken bodily measurements. In other words, the head-mounted augmented reality glasses 110 are configured to show, through dotted outline virtual overlays 122 and 124 (Figure 9) as viewed by the rescuer through the glasses 110, the exact place and orientation on the victim's chest for the first responder to place the AED electrodes 108 and 106, respectively.

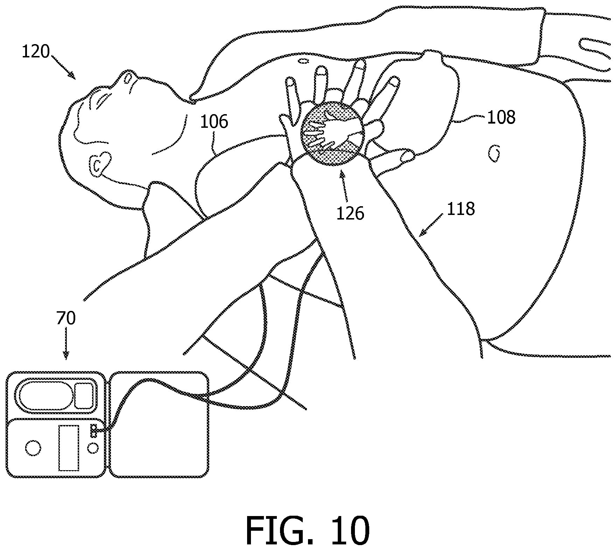

With reference now to Figures 7, 8 and 10, in one embodiment, the head-mounted augmented reality headset 110 is configured to detect the victim's face and takes into account the distance eyes-sternum to indicate where the first responder 118 should put his or her hands on the chest of the victim 120 and apply pressure during the emergency treatment. For example, the portable medical device 70 accesses the victim's cloud-based medical record, as discussed herein, and obtains, i.e., downloads, the previously taken measurement of the distance from eyes to sternum from the victim's online record. Accordingly, the head-mounted augmented reality headset 110 is used for guiding a placement of the user's hands for administering CPR which takes into account the distance eyes-sternum of the victim as provided in the real-time treatment guidance to the first responder or rescuer 118 during the emergency treatment. In this embodiment, the head-mounted augmented reality headset 110 points out the exact place for the first responder to put his or her hands, based on the previously taken measurement of the distance from eyes to sternum. In other words, the head-mounted augmented reality glasses 110 are configured to show, through a virtual overlay 126 (Figure 10) as viewed by the rescuer through the glasses 110, the exact place on the victim's chest for the first responder to put his or her hands.

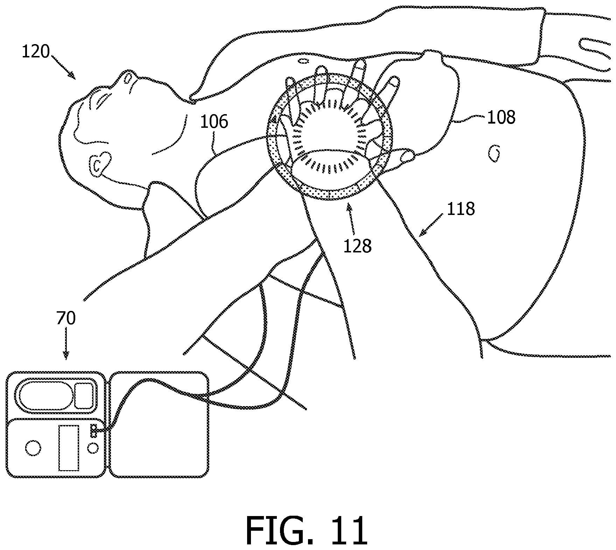

With reference now to Figures 7, 8 and 11, in one embodiment, the head-mounted augmented reality glasses 110 are configured to show, through an animated virtual overlay 128 as viewed by the rescuer through the glasses 110, how deep for the rescuer 118 to press upon the victim's chest during the emergency treatment, i.e., CPR. In addition, one or more of the outer and inner rings in the overlay 128 can comprise a form of metronome, i.e., it not only guides the user for optimal placement of the hands, but also indicates by means of animation, the optimal rhythm/speed of compression. The rhythm/speed of compression comprises, for example, a given number of compressions/minute. These settings may comprise a real-time individual treatment guidance that is optimal for the particular victim, which is the basis for adjusting the visual displays. In addition, the real-time individual treatment guidance may be provided to audible and/or visual user prompts that are issued by the CPR metronome 114 or a defibrillator.

In yet another embodiment, the CPR metronome 114 may also include a physical measuring tool which would enable a rescuer 118 to compare the eyes-'CPR device' distance with the optimal eyes-sternum distance as included in the cloud-based electronic health record. Such a comparison could be used with the real-time treatment guidance to indicate where the first responder or rescuer 118 should put his or her hands on the victim's chest and apply pressure, similar to the hands placement as shown in Figure 10.

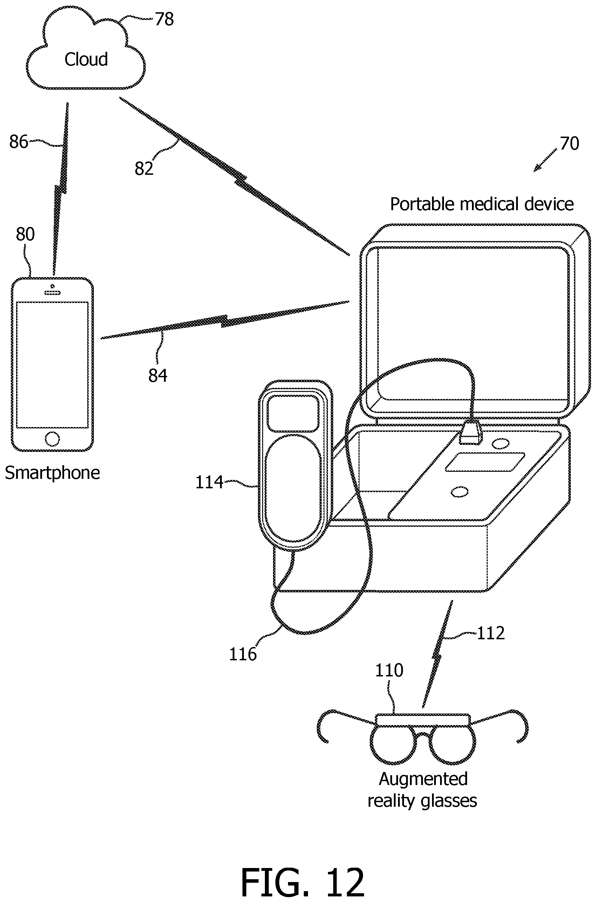

With reference now to Figure 12, a block diagram view of a portable medical apparatus 70 for real-time individualized treatment guidance for a subject according to another embodiment of the present disclosure is shown. The portable medical apparatus 70 is similar to that as shown in Figure 7, except for the following differences. The portable medical apparatus does not include components for performing AED. In addition, the CPR metronome 114 is operatively coupled to the portable medical apparatus via a wired connection 116. Furthermore, operation of the portable medical apparatus 70 of Figure 12 for real-time treatment guidance is similar to that as previously discussed herein in connection with CPR emergency treatment.

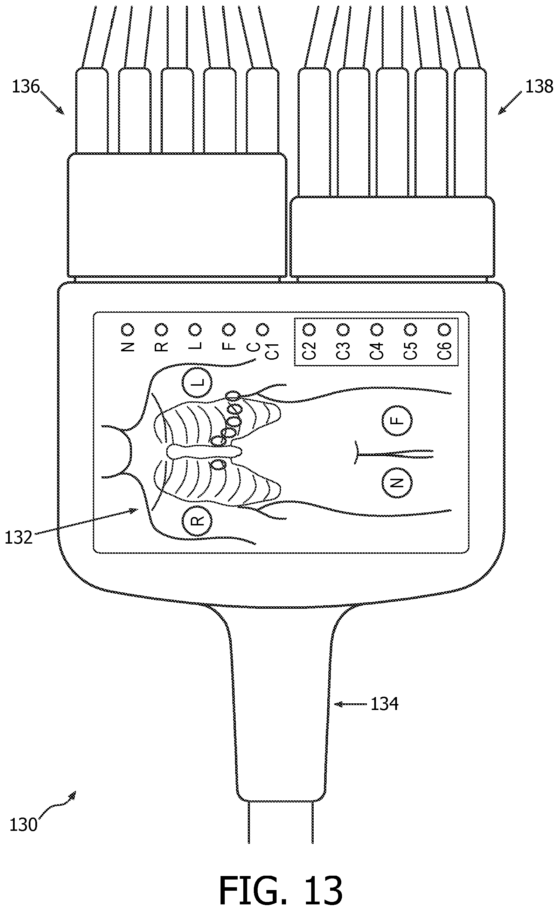

Turning now to Figure 13, an image view of an ECG lead unit interface box 130 for a portable medical apparatus, similar to the embodiments as discussed herein, having indications thereon that feature a general representation 132 for an approximate placement of electrodes on a subject's body. The interface box 130 includes a first cable 134 for operatively coupling with the portable medical apparatus, and further having cables generally indicated by reference numerals 136 and 138) with electrode pads (not shown) at distal ends thereof for use by a care provider for real-time individualized treatment guidance for the subject, according to an embodiment of the present disclosure. By itself, the general representation 132 on the interface box 130 could be confusing to the care provider placing the electrodes on a subject, i.e., absent the real-time treatment guidance provided according to the embodiments of the present disclosure.

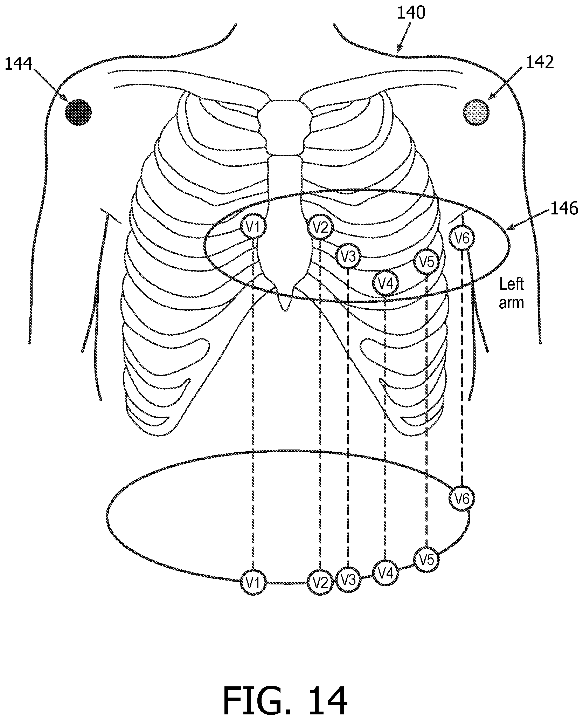

In connection with the ECG lead unit interface box 130 of Figure 13, and with additional reference to Figure 14, an illustrative diagram view is shown of ECG electrode placement on a subject's body, generally indicated by reference numeral 140. Without real-time treatment guidance, it is easy to understand that ECG electrode placement is error prone. As illustrated in Figure 14, one electrode 142 (also referred to as electrode LA) is for placement on the subject's left arm between the shoulder and the elbow, another electrode 144 (also referred to as electrode RA) is for placement on the subject's right arm between the shoulder and the elbow, and six electrodes (V1, V2, V3, V4, V5 and V6), collectively indicated by reference numeral 146, are for specific placement about the subject's rib cage. Two additional electrodes (also referred to as RL and LL, not shown) are for placement on the subject's thighs, above the ankle and below the torso.

The embodiments of the present disclosure advantageously eliminate and/or significantly diminish potential problems of electrode misplacement and electrode reversal in connection with an ECG. With respect to electrode misplacement, absent real-time treatment guidance according to the embodiments of the present disclosure, up to 50% of cases have the V1 and V2 electrodes in a more superior location, which can mimic an anterior myocardial infarction (MI) and cause T-wave inversion (i.e., negative T-waves, wherein T-wave represents the repolarization (or recovery) of the heart's ventricles). In addition, up to 33% of cases have the precordial electrodes (V1-V6) inferiorly or laterally misplaced, which can alter the amplitude and lead to a misdiagnosis. With respect to electrode reversal, absent real-time treatment guidance according to the embodiments of the present disclosure, the following scenarios can occur: RA/LA reversal - Lead I is inverted, Lead II and III are reversed, aVR and aVL are reversed; RA/LA reversal - Lead II shows isolated asystole, aVF and aVR are identical; and LA/LL Reversal - Lead III is inverted, aVL and aVF are reversed. The ECG leads are grouped into two electrical planes. The frontal leads (Lead I-III, aVR-F) view the heart from a vertical plane, while the transverse leads (V1-V6) view the heart from a horizontal plane. As used herein with reference to ECG, a lead is a view of the electrical activity of the heart from a particular angle across the body. In other words, a lead is a picture that is captured by a group of electrodes.

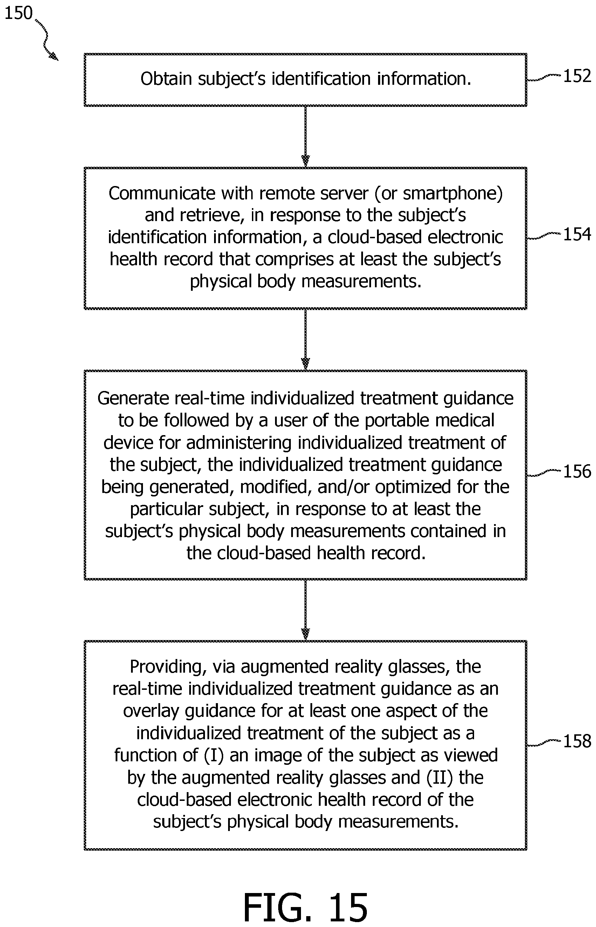

With reference now to Figure 15, there is shown a flow diagram view of a method 150, according to another embodiment, for real-time individualized treatment guidance for a subject using a portable medical device. Some aspects of the method as shown in Figure 15 are similar to and/or in addition to those discussed herein with reference to Figure 6. The method 150 comprises: obtaining 152, via a user interface, the subject's identification information; communicating 154, via a communications module, with at least one of (i) a remote server having stored thereon a cloud-based electronic health record that comprises at least the subject's physical body measurements and (ii) a smartphone located within a given immediate proximity of the medical device, wherein communicating further comprises retrieving, via at least one of the remote server or the smartphone communicating with, or the smartphone having previously communicated with, the remote server, in response to the subject's identification information, the cloud-based electronic health record; and generating 156, via a controller, real-time individualized treatment guidance to be followed by a user within the given immediate proximity of the portable medical device for administering the individualized treatment to the subject, wherein the real-time individualized treatment guidance is generated, modified and/or optimized for the particular subject, in response to at least the subject's physical body measurements from the cloud-based electronic health record.

In another embodiment, generating 156 the real-time individualized treatment guidance comprises generating at least one of a visual and audio identification of placement, as a function of the subject's physical body measurements from the cloud-based electronic health record, of defibrillation electrodes on the subject for administering a defibrillation shock. In a further embodiment, generating 156 the real-time individualized treatment guidance comprises generating at least one of a visual and audio identification of placement, as a function of the subject's physical body measurements from the cloud-based electronic health record, of hands or a cardio-pulmonary resuscitation (CPR) assistance device on the subject for administering CPR. In yet another embodiment, generating 156 the real-time individualized treatment guidance comprises generating at least one of a visual and audio identification of placement, as a function of the subject's physical body measurements from the cloud-based electronic health record, of electrocardiograph (ECG) electrodes or sensors on the subject for obtaining ECG data.

In one embodiment, obtaining 152, via the user interface, comprises obtaining via at least one of a touch screen display, an optical display, a microphone, a keypad, a keyboard, a pointing device, an image capture device, a video camera, an audio output device, and an input/output device.

In another embodiment, the user interface further comprises augmented reality glasses and the method further comprises providing 158, via the augmented reality glasses, the real-time individualized treatment guidance as an overlay guidance for at least one aspect of the individualized treatment of the subject as a function of (i) an image of the subject as viewed by the augmented reality glasses and (ii) the cloud-based electronic health record of the subject's physical body measurements. The overlay guidance further comprises at least one of (i) an outline of an individualized CPR treatment location on the subject's chest, (ii) an outline illustrating a deployment of individualized compression depth and release on the subject's chest, and (iii) an outline of individualized defibrillation electrode positioning on the subject's chest. In yet another embodiment, the overlay guidance comprises an outline of individualized placement of ECG electrodes on an upper portion of the subject's body.

According to another embodiment of the present disclosure, a guidance providing portable medical device actively adjusts its compression depth guidance settings based on the victim's bodily dimensions, wherein the bodily dimensions are obtained via an online or cloud-based personal profile. In another embodiment, the portable medical device connects to the Internet for obtaining the online personal profile and provides treatment guidance which differs between persons with different body measurements.

The embodiments of the present disclosure have applicability to cardiopulmonary resuscitation (CPR) smartphone apps, augmented reality systems for emergency care, CPR metronomes, automated external defibrillators (AED's), and the like.

Although only a few exemplary embodiments have been described in detail above, those skilled in the art will readily appreciate that many modifications are possible in the exemplary embodiments without materially departing from the novel teachings and advantages of the embodiments of the present disclosure. For example, the embodiments of the present disclosure can be advantageously used in non-emergency applications, for example, in an electrocardiograph for obtaining an electrocardiogram (ECG). Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements, and/or by means of a suitably programmed computer. In a device claim enumerating several means, several of these means may be embodied by one and the same item of hardware. The mere fact that certain measures are recited in mutually different dependent claims does not indicate that a combination of these measures cannot be used to an advantage.

## Claims

1. A portable medical apparatus (70) for real-time individualized treatment guidance for a subject (120), the apparatus comprising:
a user interface (72) for obtaining the subject's identification information;
a communications module (74) configured for communicating with at least one of (i) a remote server (78) having stored thereon a cloud-based electronic health record that comprises at least the subject's physical body measurements and (ii) a smartphone (80) located within a given immediate proximity of the portable medical apparatus, the communications module (74) further for retrieving, via at least one of the remote server (78) or the smartphone (80), in response to the subject's identification information, the cloud-based electronic health record; and
a controller (76) configured for generating, in response to at least the subject's physical body measurements from the cloud-based electronic health record, real-time individualized treatment guidance to be followed by a user (118) of the portable medical apparatus for administering the individualized treatment to the subject (120),
wherein the user interface (72) further comprises:
augmented reality glasses (110) configured to provide the real-time individualized treatment guidance as an overlay guidance for at least one aspect of the individualized treatment of the subject as a function of (i) an image of the subject (120) as viewed by the augmented reality glasses and (ii) the cloud-based electronic health record of the subject's physical body measurements.

2. The apparatus (70) of claim 1, wherein the user interface (72) comprises at least one of a touch screen display, an optical display, a microphone, a keypad, a keyboard, a pointing device, an image capture device, a video camera, an audio output device, and an input/output device.

3. The apparatus (70) of claim 1, wherein the real-time individualized treatment guidance comprises at least one of a visual and audio identification of placement, as a function of the subject's physical body measurements from the cloud-based electronic health record, of defibrillation electrodes (106,108) on the subject (120) for administering a defibrillation shock.

4. The apparatus (70) of claim 1, wherein the real-time individualized treatment guidance comprises at least one of a visual and audio identification of placement, as a function of the subject's physical body measurements from the cloud-based electronic health record, of hands (126) or a cardio-pulmonary resuscitation (CPR) assistance device (114) on the subject (120) for administering CPR.

5. The apparatus (70) of claim 1, wherein the real-time individualized treatment guidance comprises at least one of a visual and audio identification of placement, as a function of the subject's physical body measurements from the cloud-based electronic health record, of electrocardiograph (ECG) electrodes on the subject (120) for obtaining ECG data.

6. The apparatus (70) of claim 1, wherein the overlay guidance comprises at least one of (i) an outline (126) of an individualized CPR treatment location on the subject's chest, (ii) an outline (128) illustrating a deployment of individualized compression depth and release on the subject's chest, and (iii) an outline (122,124) of individualized defibrillation electrode positioning on the subject's chest.

7. The apparatus (70) of claim 1, wherein the overlay guidance comprises an outline of individualized placement of ECG electrodes on an upper portion of the subject's body.

8. The apparatus (70) of claim 1, wherein the portable medical apparatus comprises at least one selected from the group consisting of an emergency automated external defibrillator (AED), an emergency CPR metronome, and an electrocardiograph (ECG) monitor.

9. A method (150) for real-time individualized treatment guidance for a subject (120) using a portable medical device (70), the method comprising:
obtaining (152), via a user interface (72), the subject's identification information;
communicating (154), via a communications module (74), with at least one of (i) a remote server (78) having stored thereon a cloud-based electronic health record that comprises at least the subject's physical body measurements and (ii) a smartphone (80) located within a given immediate proximity of the medical device, wherein communicating further comprises retrieving, via at least one of the remote server (78) or the smartphone (80), in response to the subject's identification information, the cloud-based electronic health record; and
generating (156), via a controller (76), real-time individualized treatment guidance to be followed by a user (118) of the portable medical device for administering the individualized treatment to the subject (120), wherein the real-time individualized treatment guidance is generated in response to at least the subject's physical body measurements from the cloud-based electronic health record, the method further comprising:
providing (158), via the augmented reality glasses (110), the real-time individualized treatment guidance as an overlay guidance for at least one aspect of the individualized treatment of the subject as a function of (i) an image of the subject (120) as viewed by the augmented reality glasses and (ii) the cloud-based electronic health record of the subject's physical body measurements.

10. The method (150) of claim 9, wherein obtaining via the user interface (72) comprises obtaining via at least one of a touch screen display, an optical display, a microphone, a keypad, a keyboard, a pointing device, an image capture device, a video camera, an audio output device, and an input/output device.

11. The method (150) of claim 9, wherein generating the real-time individualized treatment guidance comprises generating at least one of a visual and audio identification of placement, as a function of the subject's physical body measurements from the cloud-based electronic health record, of defibrillation electrodes (106,108) on the subject (120) for administering a defibrillation shock.

12. The method (150) of claim 9, wherein generating the real-time individualized treatment guidance comprises generating at least one of a visual and audio identification of placement, as a function of the subject's physical body measurements from the cloud-based electronic health record, of hands or a cardio-pulmonary resuscitation (CPR) assistance device on the subject (120) for administering CPR.

13. The method (150) of claim 9, wherein the overlay guidance further comprises at least one of (i) an outline (126) of an individualized CPR treatment location on the subject's chest, (ii) an outline (128) illustrating a deployment of individualized compression depth and release on the subject's chest, and (iii) an outline (122,124) of individualized defibrillation electrode positioning on the subject's chest.

14. The method (150) of claim 9, wherein the overlay guidance further comprises an outline of individualized placement of ECG electrodes on an upper portion of the subject's body.

15. Computer program comprising program code means for causing a computer to carry out the steps of the method (150) as claimed in claim 9 when said computer program is carried out on the computer.

## Patentansprüche

1. Tragbare medizinische Vorrichtung (70) zur individuellen Behandlungsführung für ein Subjekt (120) in Echtzeit, wobei die Vorrichtung umfasst:
eine Benutzerschnittstelle (72) zum Erhalten der Identifikationsinformationen des Subjekts;
ein Kommunikationsmodul (74), das zur Kommunikation mit mindestens einem von Folgendem konfiguriert ist: (i) einem entfernten Server (78), auf dem eine cloudbasierte elektronische Gesundheitsakte gespeichert ist, die mindestens die physischen Körpermaße des Subjekts umfasst, und (ii) einem Smartphone (80), das sich in einer gegebenen unmittelbaren Nähe der tragbaren medizinischen Vorrichtung befindet, wobei das Kommunikationsmodul (74) ferner dazu dient, die cloudbasierte elektronische Gesundheitsakte als Reaktion auf die Identifikationsinformationen des Subjekts über den entfernten Server (78) und/oder das Smartphone (80) abzurufen; und
eine Steuerung (76), die so konfiguriert ist, dass sie als Reaktion auf zumindest die physischen Körpermaße des Subjekts aus der cloudbasierten elektronischen Gesundheitsakte eine individuelle Behandlungsführung in Echtzeit erzeugt, die von einem Benutzer (118) der tragbaren medizinischen Vorrichtung befolgt werden soll, um dem Subjekt (120) die individuelle Behandlung zu verabreichen, wobei die Benutzerschnittstelle (72) ferner umfasst:
Augmented-Reality-Brille (110), die so konfiguriert ist, dass sie die individualisierte Behandlungsführung in Echtzeit als Überlagerungsführung für mindestens einen Aspekt der individualisierten Behandlung des Subjekts als Funktion von Folgendem bereitstellt: (i) ein Bild des Subjekts (120), wie es durch die Augmented-Reality-Brille betrachtet wird, und (ii) die cloudbasierte elektronische Gesundheitsakte der physischen Körpermaße des Subjekts.

2. Vorrichtung (70) nach Anspruch 1, wobei die Benutzerschnittstelle (72) mindestens eines von einem Touchscreen-Display, einem optischen Display, einem Mikrofon, einem Tastenfeld, einer Tastatur, einer Zeigevorrichtung, einer Bilderfassungsvorrichtung, einer Videokamera, einer Audioausgabevorrichtung und einer Eingabe-/Ausgabevorrichtung umfasst.

3. Vorrichtung (70) nach Anspruch 1, wobei die individuelle Behandlungsführung in Echtzeit mindestens eine visuelle und akustische Identifizierung der Platzierung von Defibrillationselektroden (106, 108) auf dem Subjekt (120) zur Verabreichung eines Defibrillationsschocks als Funktion der physischen Körpermaße des Subjekts aus der cloudbasierten elektronischen Gesundheitsakte umfasst.

4. Vorrichtung (70) nach Anspruch 1, wobei die individuelle Behandlungsführung in Echtzeit mindestens eine visuelle und akustische Identifizierung der Platzierung von Händen (126) oder einer Herz-Lungen-Wiederbelebung (HLW)-Hilfsvorrichtung (114) am Subjekt (120) zur Durchführung von HLW als Funktion der physischen Körpermaße des Subjekt aus der cloudbasierten elektronischen Gesundheitsakte umfasst.

5. Vorrichtung (70) nach Anspruch 1, wobei die individuelle Behandlungsführung in Echtzeit mindestens eine visuelle und akustische Identifizierung der Platzierung von Elektrokardiograph(EKG)-Elektroden am Subjekt (120) zum Erhalten von EKG-Daten als Funktion der physischen Körpermaße des Subjekts aus der cloudbasierten elektronischen Gesundheitsakte umfasst.

6. Vorrichtung (70) nach Anspruch 1, wobei die Überlagerungsführung mindestens eines von (i) einem Umriss (126) einer individuellen HLW-Behandlungsstelle auf der Brust des Subjekts, (ii) einem Umriss (128), der den Einsatz individueller Kompressionstiefe und - entlastung auf der Brust des Subjekts veranschaulicht, und (iii) einem Umriss (122,124) der individuellen Positionierung der Defibrillationselektrode auf der Brust des Subjekts umfasst.

7. Vorrichtung (70) nach Anspruch 1, wobei die Überlagerungsführung einen Umriss der individuellen Platzierung von EKG-Elektroden auf einem oberen Teil des Körpers des Subjekts umfasst.

8. Vorrichtung (70) nach Anspruch 1, wobei die tragbare medizinische Vorrichtung mindestens eines aus der Gruppe bestehend aus einem automatisierten externen Notfall-Defibrillator (AED), einem Notfall-HLW-Metronom und einem Elektrokardiographen (EKG)-Monitor umfasst.

9. Verfahren (150) zur individuellen Behandlungsführung in Echtzeit für ein Subjekt (120) unter Verwendung einer tragbaren medizinischen Vorrichtung (70), wobei das Verfahren Folgendes umfasst:
Erhalten (152) der Identifikationsinformationen des Subjekts über eine Benutzerschnittstelle (72);
Kommunizieren (154) über ein Kommunikationsmodul (74) mit mindestens einem von (i) einem entfernten Server (78), auf dem eine cloudbasierte elektronische Gesundheitsakte gespeichert ist, die mindestens die physischen Körpermaße des Subjekts umfasst, und (ii) einem Smartphone (80), das sich in einer gegebenen unmittelbaren Nähe der medizinischen Vorrichtung befindet, wobei das Kommunizieren weiterhin das Abrufen der cloudbasierten elektronischen Gesundheitsakte als Reaktion auf die Identifikationsinformationen des Subjekts über den entfernten Server (78) und/oder das Smartphone (80) umfasst; und
Erzeugen (156) über eine Steuerung (76) einer individualisierten Behandlungsführung in Echtzeit, die von einem Benutzer (118) der tragbaren medizinischen Vorrichtung befolgt werden soll, um dem Subjekt (120) die individualisierte Behandlung zu verabreichen, wobei die individuelle Behandlungsführung in Echtzeit als Reaktion auf mindestens die physischen Körpermaße des Subjekts aus der cloudbasierten elektronischen Gesundheitsakte generiert wird, wobei das Verfahren weiterhin Folgendes umfasst:
Bereitstellen (158) der individualisierten Behandlungsführung in Echtzeit über die Augmented-Reality-Brille (110) als Überlagerungsführung für mindestens einen Aspekt der individualisierten Behandlung des Subjekts als Funktion von (i) einem Bild des Subjekts (120), wie es durch die Augmented-Reality-Brille betrachtet wird, und (ii) der cloudbasierten elektronischen Gesundheitsakte der physischen Körpermaße des Subjekts.

10. Verfahren (150) nach Anspruch 9, wobei das Erhalten über die Benutzerschnittstelle (72) das Erhalten über mindestens eines von einem Touchscreen-Display, einem optischen Display, einem Mikrofon, einem Tastenfeld, einer Tastatur, einer Zeigevorrichtung, einer Bilderfassungsvorrichtung, einer Videokamera, einer Audioausgabevorrichtung und einer Eingabe-/Ausgabevorrichtung umfasst.

11. Verfahren (150) nach Anspruch 9, wobei das Erzeugen der individuellen Behandlungsführung in Echtzeit das Erzeugen mindestens einer visuellen und akustischen Identifizierung der Platzierung von Defibrillationselektroden (106, 108) an dem Subjekt (120) zur Verabreichung eines Defibrillationsschocks als Funktion der physischen Körpermaße des Subjekts aus der cloudbasierten elektronischen Gesundheitsakte umfasst.

12. Verfahren (150) nach Anspruch 9, wobei das Erzeugen der individuellen Behandlungsführung in Echtzeit das Erzeugen mindestens einer visuellen und akustischen Identifizierung der Platzierung von Händen oder einer Herz-Lungen-Wiederbelebung (HLW)-Hilfsvorrichtung am Subjekt (120) zur Durchführung von HLW als Funktion der physischen Körpermaße des Subjekt aus der cloudbasierten elektronischen Gesundheitsversorgung umfasst.

13. Verfahren (150) nach Anspruch 9, wobei die Überlagerungsführung außerdem mindestens eines von (i) einem Umriss (126) einer individuellen HLW-Behandlungsstelle auf der Brust des Subjekt, (ii) einem Umriss (128), der den Einsatz einer individuellen Kompressionstiefe und -entlastung auf der Brust des Subjekts veranschaulicht, und (iii) einem Umriss (122,124) der individuellen Positionierung der Defibrillationselektrode auf der Brust des Subjekt umfasst.

14. Verfahren (150) nach Anspruch 9, wobei die Überlagerungsführung außerdem einen Umriss der individuellen Platzierung von EKG-Elektroden auf einem oberen Teil des Körpers des Subjekts umfasst.

15. Computerprogramm, das Programmcodemittel umfasst, um einen Computer zu veranlassen, die Schritte des Verfahrens (150) nach Anspruch 9 auszuführen, wenn das Computerprogramm auf dem Computer ausgeführt wird.

## Revendications

1. Appareil médical portatif (70) pour un guidage de traitement individualisé en temps réel pour un sujet (120), l'appareil comprenant:
une interface utilisateur (72) pour obtenir les informations d'identification du sujet;
un module de communication (74) configuré pour communiquer avec au moins l'un parmi (i) un serveur distant (78) sur lequel est stocké un dossier médical électronique basé sur le cloud qui comprend au moins les mesures corporelles physiques du sujet et (ii) un téléphone intelligent (80) situé à une proximité immédiate donnée de l'appareil médical portatif, le module de communication (74) étant en outre configuré pour récupérer, via au moins l'un entre le serveur distant (78) ou le téléphone intelligent (80), en réponse aux informations d'identification du sujet, le dossier médical électronique basé sur le cloud; et
un dispositif de commande (76) configuré pour générer, en réponse au moins aux mesures corporelles physiques du sujet à partir du dossier médical électronique basé sur le cloud, un guidage de traitement individualisé en temps réel à suivre par un utilisateur (118) de l'appareil médical portatif pour administrer le traitement individualisé du sujet (120),
où l'interface utilisateur (72) comprend en outre:
des lunettes de réalité augmentée (110) configurées pour fournir le guidage de traitement individualisé en temps réel en tant que guidage en surimpression pour au moins un aspect du traitement individualisé du sujet en fonction (i) d'une image du sujet (120) tel que vu par les lunettes de réalité augmentée et (ii) du dossier médical électronique basé sur le cloud des mesures corporelles physiques du sujet.

2. Appareil (70) selon la revendication 1, dans lequel l'interface utilisateur (72) comprend au moins l'un parmi un écran d'affichage tactile, un afficheur optique, un microphone, un clavier numérique, un clavier, un dispositif de pointage, un dispositif de capture d'images, une caméra vidéo, un dispositif de sortie audio, et un dispositif d'entrée/de sortie.

3. Appareil (70) selon la revendication 1, dans lequel le guidage de traitement individualisé en temps réel comprend au moins l'une entre une identification visuelle et une identification audio du placement, en fonction des mesures corporelles physiques du sujet à partir du dossier médical électronique basé sur le cloud, d'électrodes de défibrillation (106, 108) sur le sujet (120) pour administrer un choc de défibrillation.

4. Appareil (70) selon la revendication 1, dans lequel le guidage de traitement individualisé en temps réel comprend au moins l'une entre une identification visuelle et une identification audio du placement, en fonction des mesures corporelles physiques du sujet à partir du dossier médical électronique basé sur le cloud, des mains (126) ou d'un dispositif d'assistance à la réanimation cardio-pulmonaire (RCP) (114) sur le sujet (120) pour administrer la RCP.

5. Appareil (70) selon la revendication 1, dans lequel le guidage de traitement individualisé en temps réel comprend au moins l'une entre une identification visuelle et une identification audio du placement, en fonction des mesures corporelles physiques du sujet à partir du dossier médical électronique basé sur le cloud, d'électrodes d'électrocardiographe (ECG) sur le sujet (120) pour obtenir des données ECG.

6. Appareil (70) selon la revendication 1, dans lequel le guidage en surimpression comprend au moins l'un parmi (i) un contour (126) d'un emplacement de traitement de RCP individualisé sur la poitrine du sujet, (ii) un contour (128) illustrant un déploiement d'une profondeur de compression et d'une libération individualisées sur la poitrine du sujet, et (iii) un contour (122, 124) du positionnement individualisé d'électrodes de défibrillation sur la poitrine du sujet.

7. Appareil (70) selon la revendication 1, dans lequel le guidage en surimpression comprend un contour de placement individualisé d'électrodes ECG sur une partie supérieure du corps du sujet.

8. Appareil (70) selon la revendication 1, dans lequel l'appareil médical portatif comprend au moins un appareil sélectionné dans le groupe constitué d'un défibrillateur externe automatisé (DEA) d'urgence, d'un métronome de RCP d'urgence, et d'un moniteur d'électrocardiographe (ECG).

9. Procédé (150) pour un guidage de traitement individualisé en temps réel pour un sujet (120) en utilisant un dispositif médical portatif (70), le procédé consistant à:
obtenir (152), via une interface utilisateur (72), les informations d'identification du sujet;
communiquer (154), via un module de communication (74), avec au moins l'un parmi (i) un serveur distant (78) sur lequel est stocké un dossier médical électronique basé sur le cloud qui comprend au moins les mesures corporelles physiques du sujet et (ii) un téléphone intelligent (80) situé à une proximité immédiate donnée de l'appareil médical portatif, où la communication comprend en outre la récupération, via au moins l'un entre le serveur distant (78) ou le téléphone intelligent (80), en réponse aux informations d'identification du sujet, du dossier médical électronique basé sur le cloud; et
générer (156), via un dispositif de commande (76), un guidage de traitement individualisé en temps réel à suivre par un utilisateur (118) du dispositif médical portatif pour administrer le traitement individualisé au sujet (120), où le guidance du traitement individualisé en temps réel est généré en réponse au moins aux mesures corporelles physiques du sujet à partir du dossier médical électronique basé sur le cloud, le procédé consistant en outre à:
fournir (158), via les lunettes de réalité augmentée (110), le guidage de traitement individualisé en temps réel en tant que guidage en surimpression pour au moins un aspect du traitement individualisé du sujet en fonction (i) d'une image du sujet (120) tel que vu par les lunettes de réalité augmentée et (ii) du dossier médical électronique basé sur le cloud des mesures corporelles physiques du sujet.

10. Procédé (150) selon la revendication 9, dans lequel l'obtention via l'interface utilisateur (72) comprend l'obtention via au moins l'un parmi un écran d'affichage tactile, un afficheur optique, un microphone, un clavier numérique, un clavier, un dispositif de pointage, un dispositif de capture d'images, une caméra vidéo, un dispositif de sortie audio, et un dispositif d'entrée/de sortie.

11. Procédé (150) selon la revendication 9, dans lequel la génération du guidage de traitement individualisé en temps réel comprend la génération d'au moins une entre une identification visuelle et une identification audio du placement, en fonction des mesures corporelles physiques du sujet à partir du dossier médical électronique basé sur le cloud, d'électrodes de défibrillation (106, 108) sur le sujet (120) pour administrer un choc de défibrillation.

12. Procédé (150) selon la revendication 9, dans lequel la génération du guidage de traitement individualisé en temps réel comprend la génération d'au moins une entre une identification visuelle et une identification audio du placement, en fonction des mesures corporelles physiques du sujet à partir du dossier médical électronique basé sur le cloud, des mains ou d'un dispositif d'assistance à la réanimation cardio-pulmonaire (RCP) sur le sujet (120) pour administrer la RCP.

13. Procédé (150) selon la revendication 9, dans lequel le guidage en surimpression comprend en outre au moins l'un parmi (i) un contour (126) d'un emplacement de traitement de RCP individualisé sur la poitrine du sujet, (ii) un contour (128) illustrant un déploiement d'une profondeur de compression et d'une libération individualisées sur la poitrine du sujet, et (iii) un contour (122, 124) du positionnement individualisé de l'électrode de défibrillation sur la poitrine du sujet.

14. Procédé (150) selon la revendication 9, dans lequel le guidage en surimpression comprend en outre un contour de placement individualisé d'électrodes ECG sur une partie supérieure du corps du sujet.

15. Programme informatique comprenant des moyens de code de programme pour amener un ordinateur à exécuter les étapes du procédé (150) selon la revendication 9 lorsque ledit programme informatique est exécuté sur l'ordinateur.
